# EUROPEAN PATENT APPLICATION

(11) **EP 2 154 535 A1**
(43) Date of publication of application: **17.02.2010**
(21) Application number: 09167667.6
(22) Date of filing: 09.12.1999
(51) Int. Cl.: G01N 33/536, C07K 14/00, C12N 15/00, C12N 15/10

(54) **Protein scaffolds for antibody mimics and other binding proteins**

(30) Priority: 10.12.1998 US 111737 P
(62) Divisional of application: 99967261.1
(71) Applicant: Bristol-Myers Squibb Company, Princeton, NJ 05843-4000 (US)
(72) Inventor: Lipovsek, Dasa, Cambridge, MA 02138 (US)
(74) Representative: Reitstötter - Kinzebach

(57) **Abstract**

Disclosed herein are proteins that include a fibronectin type III domain having at least one randomized loop. Also disclosed herein are nucleic acids encoding such proteins and the use of such proteins in methods for evolving novel compound-binding species and their ligands.

## Description

### Background of the Invention

This invention relates to protein scaffolds useful, for example, for the generation of products having novel binding characteristics.

Proteins having relatively defined three-dimensional structures, commonly referred to as protein scaffolds, may be used as reagents for the design of engineered products. These scaffolds typically contain one or more regions which are amenable to specific or random sequence variation, and such sequence randomization is often carried out to produce libraries of proteins from which desired products may be selected. One particular area in which such scaffolds are useful is the field of antibody design.

A number of previous approaches to the manipulation of the mammalian immune system to obtain reagents or drugs have been attempted. These have included injecting animals with antigens of interest to obtain mixtures of polyclonal antibodies reactive against specific antigens, production of monoclonal antibodies in hybridoma cell culture (Koehler and Milstein, Nature 256:495, 1975), modification of existing monoclonal antibodies to obtain new or optimized recognition properties, creation of novel antibody fragments with desirable binding characteristics, and randomization of single chain antibodies (created by connecting the variable regions of the heavy and light chains of antibody molecules with a flexible peptide linker) followed by selection for antigen binding by phage display (Clackson et al., Nature 352:624, 1991).

In addition, several non-immunoglobulin protein scaffolds have been proposed for obtaining proteins with novel binding properties. For example, a "minibody" scaffold, which is related to the immunoglobulin fold, has been designed by deleting three beta strands from a heavy chain variable domain of a monoclonal antibody (Tramontano et al., J. Mol. Recognit. 7:9, 1994). This protein includes 61 residues and can be used to present two hypervariable loops. These two loops have been randomized and products selected for antigen binding, but thus far the framework appears to have somewhat limited utility due to solubility problems. Another framework used to display loops has been tendamistat, a 74 residue, six-strand beta sheet sandwich held together by two disulfide bonds (McConnell and Hoess, J. Mol. Biol. 250:460, 1995). This scaffold includes three loops, but, to date, only two of these loops have been examined for randomization potential.

Other proteins have been tested as frameworks and have been used to display randomized residues on alpha helical surfaces (Nord et al., Nat. Biotechnol. 15:772, 1997; Nord et al., Protein Eng. 8:601, 1995), loops between alpha helices in alpha helix bundles (Ku and Schultz, Proc. Natl. Acad. Sci. USA 92:6552, 1995), and loops constrained by disulfide bridges, such as those of the small protease inhibitors (Markland et al., Biochemistry 35:8045, 1996; Markland et al., Biochemistry 35:8058, 1996; Rottgen and Collins, Gene 164:243, 1995; Wang et al., J. Biol. Chem. 270:12250, 1995).

### Summary of the Invention

The present invention provides a new family of proteins capable of evolving to bind any compound of interest. These proteins, which make use of a fibronectin or fibronectin-like scaffold, function in a manner characteristic of natural or engineered antibodies (that is, polyclonal, monoclonal, or single-chain antibodies) and, in addition, possess structural advantages. Specifically, the structure of these antibody mimics has been designed for optimal folding, stability, and solubility, even under conditions which normally lead to the loss of structure and function in antibodies.

These antibody mimics may be utilized for the purpose of designing proteins which are capable of binding to virtually any compound (for example, any protein) of interest. In particular, the fibronectin-based molecules described herein may be used as scaffolds which are subjected to directed evolution designed to randomize one or more of the three fibronectin loops which are analogous to the complementarity-determining regions (CDRs) of an antibody variable region. Such a directed evolution approach results in the production of antibody-like molecules with high affinities for antigens of interest. In addition, the scaffolds described herein may be used to display defined exposed loops (for example, loops previously randomized and selected on the basis of antigen binding) in order to direct the evolution of molecules that bind to such introduced loops. A selection of this type may be carried out to identify recognition molecules for any individual CDR-like loop or, alternatively, for the recognition of two or all three CDR-like loops combined into a non-linear epitope.

Accordingly, the present invention features a protein that includes a fibronectin type III domain having at least one randomized loop, the protein being characterized by its ability to bind to a compound that is not bound by the corresponding naturally-occurring fibronectin.

In preferred embodiments, the fibronectin type III domain is a mammalian (for example, a human) fibronectin type III domain; and the protein includes the tenth module of the fibronectin type III (¹⁰Fn3) domain. In such proteins, compound binding is preferably mediated by either one, two, or three ¹⁰Fn3 loops. In other preferred embodiments, the second loop of ¹⁰Fn3 may be extended in length relative to the naturally-occurring module, or the ¹⁰Fn3 may lack an integrin-binding motif. In these molecules, the integrin-binding motif may be replaced by an amino acid sequence in which a basic amino acid-neutral amino acid-acidic amino acid sequence (in the N-terminal to C-terminal direction) replaces the integrin-binding motif; one preferred sequence is serine-glycine-glutamate. In another preferred embodiment, the fibronectin type III domain-containing proteins of the invention lack disulfide bonds.

Any of the fibronectin type II domain-containing proteins described herein may be formulated as part of a fusion protein (for example, a fusion protein which further includes an immunoglobulin F_{c} domain, a complement protein, a toxin protein, or an albumin protein). In addition, any of the fibronectin type III domain proteins may be covalently bound to a nucleic acid (for example, an RNA), and the nucleic acid may encode the protein. Moreover, the protein may be a multimer, or, particularly if it lacks an integrin-binding motif, it may be formulated in a physiologically-acceptable carrier.

The present invention also includes features proteins that include a fibronectin type III domain having at least one mutation in a β-sheet sequence which changes the scaffold structure. Again, these proteins are characterized by their ability to bind to compound that are not bound by the corresponding naturally-occurring fibronectin.

In a related aspect, the invention further features nucleic acids encoding any of the proteins of the invention. In preferred embodiments, the nucleic acid is DNA or RNA.

In another related aspect, the invention also features a method for generating a protein which includes a fibronectin type III domain and which is pharmaceutically acceptable to a mammal, involving removing the integrin-binding domain of said fibronectin type III domain. This method may be applied to any of the fibronectin type III domain-containing proteins described above and is particularly useful for generating proteins for human therapeutic applications. The invention also features such fibronectin type III domain-containing proteins which lack integrin-binding domains.

In yet other related aspects, the invention features screening methods which may be used to obtain or evolve randomized fibronectin type III proteins capable of binding to compounds of interest, or to obtain or evolve compounds (for example, proteins) capable of binding to a particular protein containing a randomized fibronectin type III motif. In addition, the invention features screening procedures which combine these two methods, in any order, to obtain either compounds or proteins of interest.

In particular, the first screening method, useful for the isolation or identification of randomized proteins of interest, involves : (a) contacting the compound with a candidate protein, the candidate protein including a fibronectin type III domain having at least one randomized loop, the contacting being carried out under conditions that allow compound-protein complex formation; and (b) obtaining, from the complex, the protein which binds to the compound.

The second screening method, for isolating or identifying a compound which binds to a protein having a randomized fibronectin type III domain, involves: (a) contacting the protein with a candidate compound, the contacting being carried out under conditions that allow compound-protein complex formation; and (b) obtaining, from the complex, the compound which binds to the protein.

In preferred embodiments, the methods further involve either randomizing at least one loop of the fibronectin type III domain of the protein obtained in step (b) and repeating steps (a) and (b) using the further randomized protein, or modifying the compound obtained in step (b) and repeating steps (a) and (b) using the further modified compound. In addition, the compound is preferably a protein, and the fibronectin type III domain is preferably a mammalian (for example, a human) fibronectin type III domain. In other preferred embodiments, the protein includes the tenth module of the fibronectin type III domain (¹⁰Fn3), and binding is mediated by one, two or three ¹⁰Fn3 loops. In addition, the second loop of ¹⁰Fn3 may be extended in length relative to the naturally-occurring module, or ¹⁰Fn3 may lack an integrin-binding motif. Again, as described above, the integrin-binding motif may be replaced by an amino acid sequence in which a basic amino acid-neutral amino acid-acidic amino acid sequence (in the N-terminal to C-terminal direction) replaces the integrin-binding motif; one preferred sequence is serine-glycine-glutamate.

The selection methods described herein may be carried out using any fibronectin type III domain-containing protein. For example, the fibronectin type III domain-containing protein may lack disulfide bonds, or may be formulated as part of a fusion protein (for example, a fusion protein which further includes an immunoglobulin F_{c} domain, a complement protein, a toxin protein, or an albumin protein). In addition, selections may be carried out using the fibronectin type III domain proteins covalently bound to nucleic acids (for example, RNAs or any nucleic acid which encodes the protein). Moreover, the selections may be carried out using fibronectin domain-containing protein multimers.

Preferably, the selections involve the immobilization of the binding target on a solid support. Preferred solid supports include columns (for example, affinity columns, such as agarose columns) or microchips.

As used herein, by "fibronectin type III domain" is meant a domain having 7 or 8 beta strands which are distributed between two beta sheets, which themselves pack against each other to form the core of the protein, and further containing loops which connect the beta strands to each other and are solvent exposed. There are at least three such loops at each edge of the beta sheet sandwich, where the edge is the boundary of the protein perpendicular to the direction of the beta strands. Preferably, a fibronectin type III domain includes a sequence which exhibits at least 30% amino acid identity, and preferably at least 50% amino acid identity, to the sequence encoding the structure of the ¹⁰Fn3 domain referred to as "1ttg" (ID = "1ttg" (one ttg)) available from the Protein Data Base. Sequence identity referred to in this definition is determined by the Homology program, available from Molecular Simulation (San Diego, CA). The invention further includes polymers of ¹⁰Fn3-related molecules, which are an extension of the use of the monomer structure, whether or not the subunits of the polyprotein are identical or different in sequence.

By "naturally occurring fibronectin" is meant any fibronectin protein that is encoded by a living organism.

By "randomized" is meant including one or more amino acid alterations relative to a template sequence.

By a "protein" is meant any sequence of two or more amino acids, regardless of length, post-translation modification, or function. "Protein" and "peptide" are used interchangeably herein.

By "RNA" is meant a sequence of two or more covalently bonded, naturally occurring or modified ribonucleotides. One example of a modified RNA included within this term is phosphorothioate RNA.

By "DNA" is meant a sequence of two or more covalently bonded, naturally occurring or modified deoxyribonucleotides.

By a "nucleic acid" is meant any two or more covalently bonded nucleotides or nucleotide analogs or derivatives. As used herein, this term includes, without limitation, DNA, RNA, and PNA.

By "pharmaceutically acceptable" is meant a compound or protein that may be administered to an animal (for example, a mammal) without significant adverse medical consequences.

By "physiologically acceptable carrier" is meant a carrier which does not have a significant detrimental impact on the treated host and which retains the therapeutic properties of the compound with which it is administered. One exemplary physiologically acceptable carrier is physiological saline. Other physiologically acceptable carriers and their formulations are known to one skilled in the art and are described, for example, in Remington's Pharmaceutical Sciences, (18th edition), ed. A. Gennaro, 1990, Mack Publishing Company, Easton, PA, incorporated herein by reference.

By "selecting" is meant substantially partitioning a molecule from other molecules in a population. As used herein, a "selecting" step provides at least a 2-fold, preferably, a 30-fold, more preferably, a 100-fold, and, most preferably, a 1000-fold enrichment of a desired molecule relative to undesired molecules in a population following the selection step. A selection step may be repeated any number of times, and different types of selection steps may be combined in a given approach.

By "binding partner," as used herein, is meant any molecule which has a specific, covalent or non-covalent affinity for a portion of a desired compound (for example, protein) of interest. Examples of binding partners include, without limitation, members of antigen/antibody pairs, protein/inhibitor pairs, receptor/ligand pairs (for example cell surface receptor/ligand pairs, such as hormone receptor/peptide hormone pairs), enzyme/substrate pairs (for example, kinase/substrate pairs), lectin/carbohydrate pairs, oligomeric or heterooligomeric protein aggregates, DNA binding protein/DNA binding site pairs, RNA/protein pairs, and nucleic acid duplexes, heteroduplexes, or ligated strands, as well as any molecule which is capable of forming one or more covalent or non-covalent bonds (for example, disulfide bonds) with any portion of another molecule (for example, a compound or protein).

By a "solid support" is meant, without limitation, any column (or column material), bead, test tube, microtiter dish, solid particle (for example, agarose or sepharose), microchip (for example, silicon, silicon-glass, or gold chip), or membrane (for example, the membrane of a liposome or vesicle) to which an affinity complex may be bound, either directly or indirectly (for example, through other binding partner intermediates such as other antibodies or Protein A), or in which an affinity complex may be embedded (for example, through a receptor or channel).

The present invention provides a number of advantages. For example, as described in more detail below, the present antibody mimics exhibit improved biophysical properties, such as stability under reducing conditions and solubility at high concentrations. In addition, these molecules may be readily expressed and folded in prokaryotic systems, such as E. coli, in eukaryotic systems, such as yeast, and in in vitro translation systems, such as the rabbit reticulocyte lysate system. Moreover, these molecules are extremely amenable to affinity maturation techniques involving multiple cycles of selection, including in vitro selection using RNA-protein fusion technology (Roberts and Szostak, Proc. Natl. Acad. Sci USA 94:12297, 1997; Szostak et al., U.S.S.N. 09/007,005 and U.S.S.N. 09/247,190; Szostak et al. WO98/31700), phage display (see, for example, Smith and Petrenko, Chem. Rev. 97:317, 1997), and yeast display systems (see, for example, Boder and Wittrup, Nature Biotech. 15:553, 1997).

Other features and advantages of the present invention will be apparent from the following detailed description thereof, and from the claims.

### Brief Description of the Drawings

FIGURE 1 is a photograph showing a comparison between the structures of antibody heavy chain variable regions from camel (dark blue) and llama (light blue), in each of two orientations.
FIGURE 2 is a photograph showing a comparison between the structures of the camel antibody heavy chain variable region (dark blue), the llama antibody heavy chain variable region (light blue), and a fibronectin type III module number 10 (¹⁰Fn3) (yellow).
FIGURE 3 is a photograph showing a fibronectin type III module number 10 (¹⁰Fn3), with the loops corresponding to the antigen-binding loops in IgG heavy chains highlighted in red.
FIGURE 4 is a graph illustrating a sequence alignment between a fibronectin type III protein domain and related protein domains.
FIGURE 5 is a photograph showing the structural similarities between a ¹⁰Fn3 domain and 15 related proteins, including fibronectins, tenascins, collagens, and undulin. In this photograph, the regions are labeled as follows: constant, dark blue; conserved, light blue; neutral, white; variable, red; and RGB integrin-binding motif (variable), yellow.

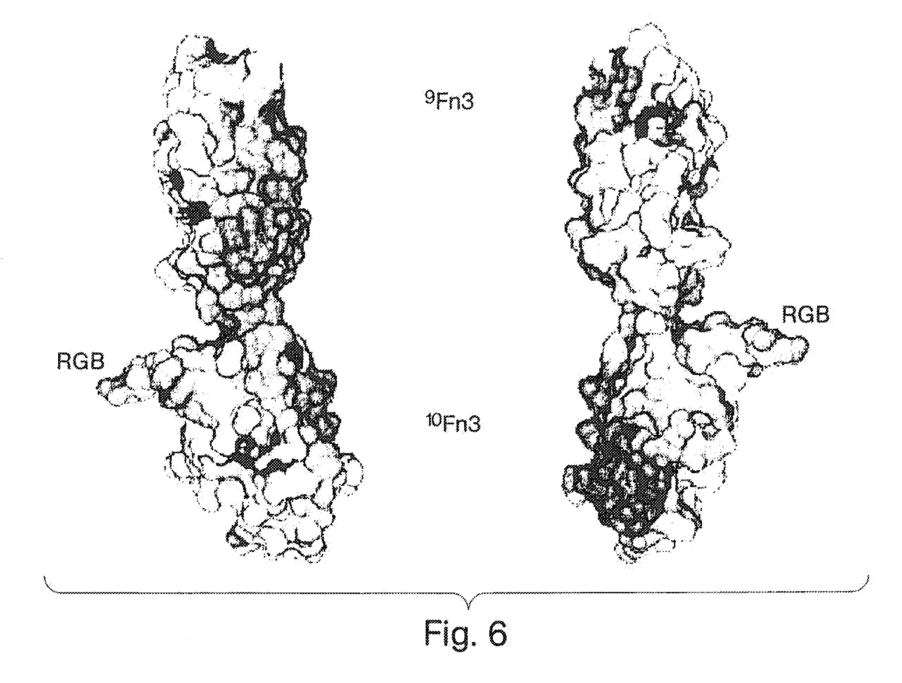

FIGURE 6 is a photograph showing space filling models of fibronectin III modules 9 and 10, in each of two different orientations. The two modules and the integrin binding loop (RGB) are labeled. In this figure, blue indicates positively charged residues, red indicates negatively charged residues, and white indicates uncharged residues.

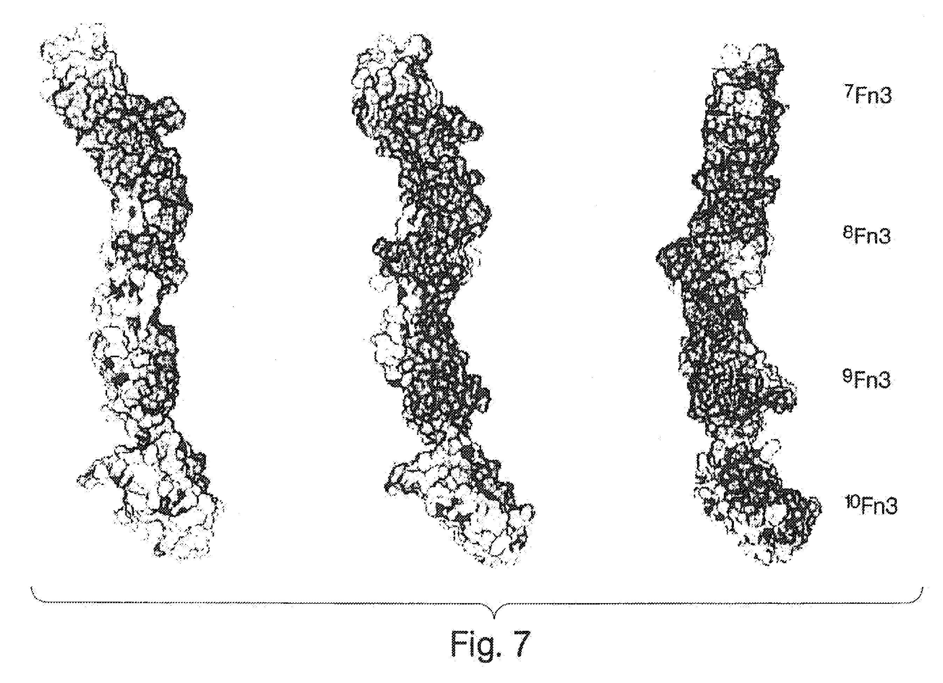

FIGURE 7 is a photograph showing space filling models of fibronectin III modules 7-10, in each of three different orientiations. The four modules are labeled. In this figure, blue indicates positively charged residues, red indicates negatively charged residues, and white indicates uncharged residues.
FIGURE 8 is a photograph illustrating the formation, under different salt conditions, of RNA-protein fusions which include fibronectin type III domains.
FIGURE 9 is a series of photographs illustrating the selection of fibronectin type III domain-containing RNA-protein fusions, as measured by PCR signal analysis.
FIGURE 10 is a graph illustrating an increase in the percent TNF-α binding during the selections described herein, as well as a comparison between RNA-protein fusion and free protein selections.
FIGURE 11 is a series of schematic representations showing IgG, ¹⁰Fn3, Fn-CH₁-CH₂-CH₃, and Fn-CH₂-CH₃ (clockwise from top left).
FIGURE 12 is a photograph showing a molecular model of Fn-CH₁-CH₂-CH₃ based on known three-dimensional structures of IgG (X-ray crystallography) and ¹⁰Fn3 (NMR and X-ray crystallography).

### Detailed Description

The novel antibody mimics described herein have been designed to be superior both to antibody-derived fragments and to non-antibody frameworks, for example, those frameworks described above.

The major advantage of these antibody mimics over antibody fragments is structural. These scaffolds are derived from whole, stable, and soluble structural modules found in human body fluid proteins. Consequently, they exhibit better folding and thermostability properties than antibody fragments, whose creation involves the removal of parts of the antibody native fold, often exposing amino acid residues that, in an intact antibody, would be buried in a hydrophobic environment, such as an interface between variable and constant domains. Exposure of such hydrophobic residues to solvent increases the likelihood of aggregation.

In addition, the antibody mimics described herein have no disulfide bonds, which have been reported to retard or prevent proper folding of antibody fragments under certain conditions. Since the present scaffolds do not rely on disulfides for native fold stability, they are stable under reducing conditions, unlike antibodies and their fragments which unravel upon disulfide bond breakdown.

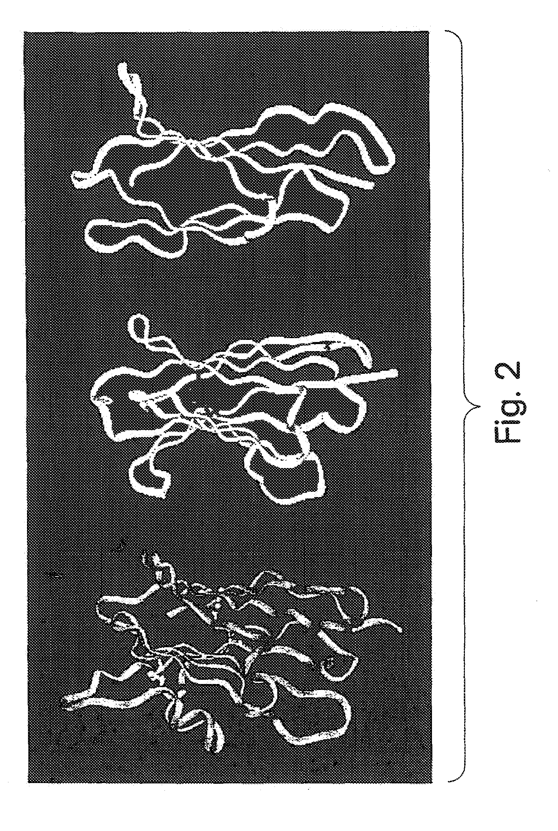

Moreover, these fibronectin-based scaffolds provide the functional advantages of antibody molecules. In particular, despite the fact that the ¹⁰Fn3 module is not an immunoglobulin, its overall fold is close to that of the variable region of the IgG heavy chain (Figure 2), making it possible to display the three fibronectin loops analogous to CDRs in relative orientations similar to those of native antibodies. Because of this structure, the present antibody mimics possess antigen binding properties that are similar in nature and affinity to those of antibodies, and a loop randomization and shuffling strategy may be employed in vitro that is similar to the process of affinity maturation of antibodies in vivo.

There are now described below exemplary fibronectin-based scaffolds and their use for identifying, selecting, and evolving novel binding proteins as well as their target ligands. These examples are provided for the purpose of illustrating, and not limiting, the invention.

### ¹⁰Fn3 Structural Motif

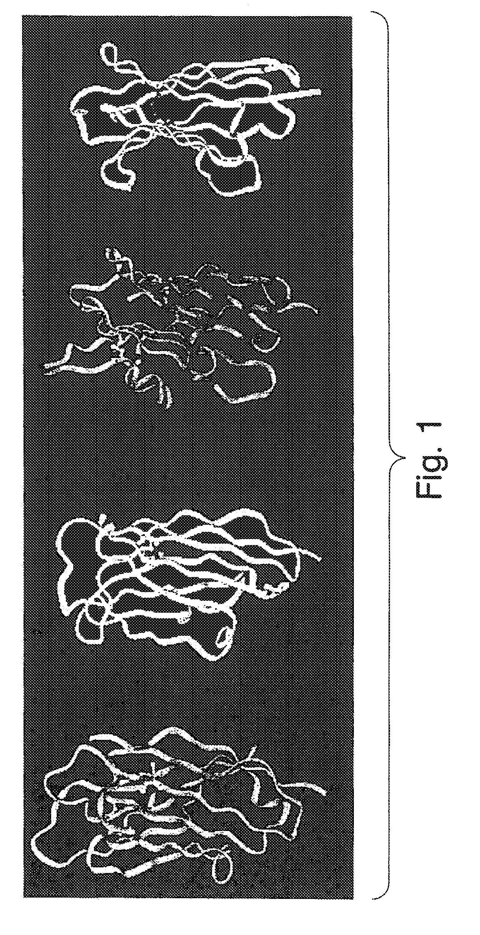

The antibody mimics of the present invention are based on the structure of a fibronectin module of type III (Fn3), a common domain found in mammalian blood and structural proteins. This domain occurs more than 400 times in the protein sequence database and has been estimated to occur in 2% of the proteins sequenced to date, including fibronectins, tenscin, intracellular cytoskeletal proteins, and prokaryotic enzymes (Bork and Doolittle, Proc. Natl. Acad. Sci. USA 89:8990, 1992; Bork et al., Nature Biotech. 15:553, 1997; Meinke et al., J. Bacteriol. 175:1910, 1993; Watanabe et al., J. Biol. Chem. 265:15659, 1990). In particular, these scaffolds include, as templates, the tenth module of human Fn3 (¹⁰Fn3), which comprises 94 amino acid residues. The overall fold of this domain is closely related to that of the smallest functional antibody fragment, the variable region of the heavy chain, which comprises the entire antigen recognition unit in camel and llama IgG (Figure 1, 2). The major differences between camel and llama domains and the ¹⁰Fn3 domain are that (i) ¹⁰Fn3 has fewer beta strands (seven vs. nine) and (ii) the two beta sheets packed against each other are connected by a disulfide bridge in the camel and llama domains, but not in ¹⁰Fn3.

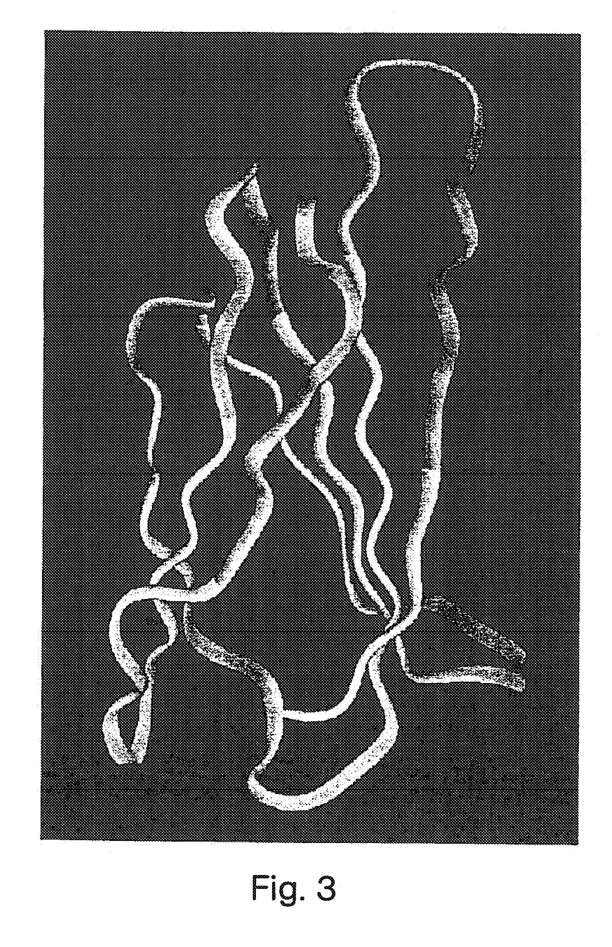

The three loops of ¹⁰Fn3 corresponding to the antigen-binding loops of the IgG heavy chain run between amino acid residues 21-31, 51-56, and 76-88 (Figure 3). The length of the first and the third loop, 11 and 12 residues, respectively, fall within the range of the corresponding antigen-recognition loops found in antibody heavy chains, that is, 10-12 and 3-25 residues, respectively. Accordingly, once randomized and selected for high antigen affinity, these two loops make contacts with antigens equivalent to the contacts of the corresponding loops in antibodies.

In contrast, the second loop of ¹⁰Fn3 is only 6 residues long, whereas the corresponding loop in antibody heavy chains ranges from 16-19 residues. To optimize antigen binding, therefore, the second loop of ¹⁰Fn3 is preferably extended by 10-13 residues (in addition to being randomized) to obtain the greatest possible flexibility and affinity in antigen binding. Indeed, in general, the lengths as well as the sequences of the CDR-like loops of the antibody mimics may be randomized during in vitro or in vivo affinity maturation (as described in more detail below).

The tenth human fibronectin type III domain, ¹⁰Fn3, refolds rapidly even at low temperature; its backbone conformation has been recovered within 1 second at 5°C. Thermodynamic stability of ¹⁰Fn3 is high (ΔG_{U} = 24 kJ/mol = 5.7 kcal/mol), correlating with its high melting temperature of 110°C.

One of the physiological roles of ¹⁰Fn3 is as a subunit of fibronectin, a glycoprotein that exists in a soluble form in body fluids and in an insoluble form in the extracellular matrix (Dickinson et al., J. Mol. Biol. 236:1079, 1994). A fibronectin monomer of 220-250 kD contains 12 type I modules, two type II modules, and 17 fibronectin type III modules (Potts and Campbell, Curr. Opin.Cell Biol. 6:648, 1994). Different type III modules are involved in the binding of fibronectin to integrins, heparin, and chondroitin sulfate. ¹⁰Fn3 was found to mediate cell adhesion through an integrin-binding Arg-Gly-Asp (RGD) motif on one of its exposed loops. Similar RGD motifs have been shown to be involved in integrin binding by other proteins, such as fibrinogen, von Wellebrand factor, and vitronectin (Hynes et al., Cell 69:11, 1992). No other matrix- or cell-binding roles have been described for ¹⁰Fn3.

The observation that ¹⁰Fn3 has only slightly more adhesive activity than a short peptide containing RGD is consistent with the conclusion that the cell-binding activity of ¹⁰Fn3 is localized in the RGD peptide rather than distributed throughout the ¹⁰Fn3 structure (Baron et al., Biochemistry 31:2068, 1992). The fact that ¹⁰Fn3 without the RGD motif is unlikely to bind to other plasma proteins or extracellular matrix makes ¹⁰Fn3 a useful scaffold to replace antibodies. In addition, the presence of ¹⁰Fn3 in natural fibrinogen in the bloodstream suggests that ¹⁰Fn3 itself is unlikely to be immunogenic in the organism of origin.

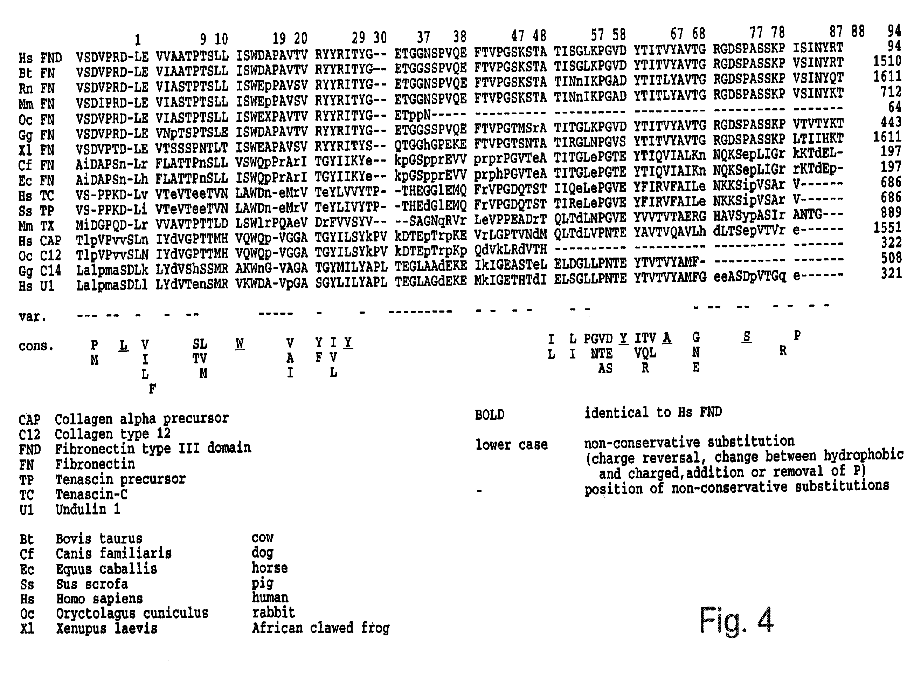

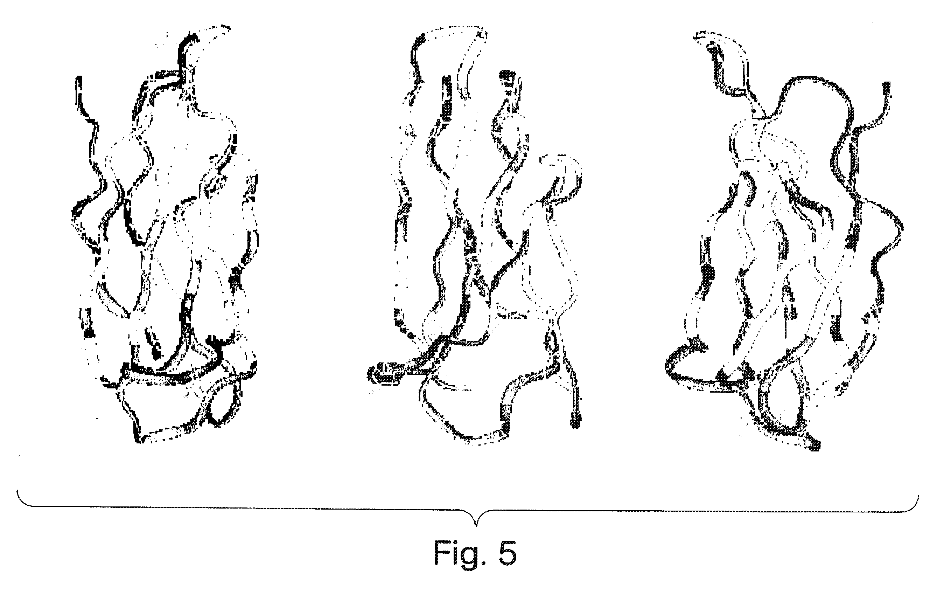

In addition, we have determined that the ¹⁰Fn3 framework possesses exposed loop sequences tolerant of randomization, facilitating the generation of diverse pools of antibody mimics. This determination was made by examining the flexibility of the ¹⁰Fn3 sequence. In particular, the human ¹⁰Fn3 sequence was aligned with the sequences of fibronectins from other sources as well as sequences of related proteins (Figure 4), and the results of this alignment were mapped onto the three-dimensional structure of the human ¹⁰Fn3 domain (Figure 5). This alignment revealed that the majority of conserved residues are found in the core of the beta sheet sandwich, whereas the highly variable residues are located along the edges of the beta sheets, including the N- and C-termini, on the solvent-accessible faces of both beta sheets, and on three solvent-accessible loops that serve as the hypervariable loops for affinity maturation of the antibody mimics. In view of these results, the randomization of these three loops are unlikely to have an adverse effect on the overall fold or stability of the ¹⁰Fn3 framework itself.

For the human ¹⁰Fn3 sequence, this analysis indicates that, at a minimum, amino acids 1-9, 44-50, 61-54, 82-94 (edges of beta sheets); 19, 21, 30-46 (even), 79-65 (odd) (solvent-accessible faces of both beta sheets); 21-31, 51-56, 76-88 (CDR-like solvent-accessible loops); and 14-16 and 36-45 (other solvent-accessible loops and beta turns) may be randomized to evolve new or improved compound-binding proteins. In addition, as discussed above, alterations in the lengths of one or more solvent exposed loops may also be included in such directed evolution methods. Alternatively, changes in the β-sheet sequences may also be used to evolve new proteins. These mutations change the scaffold and thereby indirectly alter loop structure(s). If this approach is taken, mutations should not saturate the sequence, but rather few mutations should be introduced. Preferably, no more than 10 amino acid changes, and, more preferably, no more than 3 amino acid changes should be introduced to the β-sheet sequences by this approach.

### Fibronectin Fusions

The antibody mimics described herein may be fused to other protein domains. For example, these mimics may be integrated with the human immune response by fusing the constant region of an IgG (F_{c}) with a ¹⁰Fn3 module, preferably through the C-terminus of ¹⁰Fn3. The F_{c} in such a ¹⁰Fn3-F_{c} fusion molecule activates the complement component of the immune response and increases the therapeutic value of the antibody mimic. Similarly, a fusion between ¹⁰Fn3 and a complement protein, such as C1q, may be used to target cells, and a fusion between ¹⁰Fn3 and a toxin may be used to specifically destroy cells that carry a particular antigen. In addition, ¹⁰Fn3 in any form may be fused with albumin to increase its half-life in the bloodstream and its tissue penetration. Any of these fusions may be generated by standard techniques, for example, by expression of the fusion protein from a recombinant fusion gene constructed using publically available gene sequences.

### Fibronectin Scaffold Multimers

In addition to fibronectin monomers, any of the fibronectin constructs described herein may be generated as dimers or multimers of ¹⁰Fn3-based antibody mimics as a means to increase the valency and thus the avidity of antigen binding. Such multimers may be generated through covalent binding between individual ¹⁰Fn3 modules, for example, by imitating the natural ⁸Fn3-⁹Fn3-¹⁰Fn3 C-to-N-terminus binding or by imitating antibody dimers that are held together through their constant regions. A ¹⁰Fn3-Fc construct may be exploited to design dimers of the general scheme of ¹⁰Fn3-Fc::Fc-¹⁰Fn3. The bonds engineered into the Fc::Fc interface may be covalent or non-covalent. In addition, dimerizing or multimerizing partners other than Fc can be used in ¹⁰Fn3 hybrids to create such higher order structures.

In particular examples, covalently bonded multimers may be generated by constructing fusion genes that encode the multimer or, alternatively, by engineering codons for cysteine residues into monomer sequences and allowing disulfide bond formation to occur between the expression products. Non-covalently bonded multimers may also be generated by a variety of techniques. These include the introduction, into monomer sequences, of codons corresponding to positively and/or negatively charged residues and allowing interactions between these residues in the expression products (and therefore between the monomers) to occur. This approach may be simplified by taking advantage of charged residues naturally present in a monomer subunit, for example, the negatively charged residues of fibronectin. Another means for generating non-covalently bonded antibody mimics is to introduce, into the monomer gene (for example, at the amino- or carboxy-termini), the coding sequences for proteins or protein domains known to interact. Such proteins or protein domains include coil-coil motifs, leucine zipper motifs, and any of the numerous protein subunits (or fragments thereof) known to direct formation of dimers or higher order multimers.

### Fibronectin-Like Molecules

Although ¹⁰Fn3 represents a preferred scaffold for the generation of antibody mimics, other molecules may be substituted for ¹⁰Fn3 in the molecules described herein. These include, without limitation, human fibronectin modules ¹Fn3-⁹Fn3 and ¹¹Fn3-¹⁷Fn3 as well as related Fn3 modules from non-human animals and prokaryotes. In addition, Fn3 modules from other proteins with sequence homology to ¹⁰Fn3, such as tenascins and undulins, may also be used. Modules from different organisms and parent proteins may be most appropriate for different applications; for example, in designing an antibody mimic, it may be most desirable to generate that protein from a fibronectin or fibronectin-like molecule native to the organism for which a therapeutic or diagnostic molecule is intended.

### Directed Evolution of Scaffold-Based Binding Proteins

The antibody mimics described herein may be used in any technique for evolving new or improved binding proteins. In one particular example, the target of binding is immobilized on a solid support, such as a column resin or microtiter plate well, and the target contacted with a library of candidate scaffold-based binding proteins. Such a library may consist of ¹⁰Fn3 clones constructed from the wild type ¹⁰Fn3 scaffold through randomization of the sequence and/or the length of the ¹⁰Fn3 CDR-like loops. If desired, this library may be an RNA-protein fusion library generated, for example, by the techniques described in Szostak et al., U.S.S.N. 09/007,005 and 09/247,190; Szostak et al., WO98/31700; and Roberts & Szostak, Proc. Nat1. Acad. Sci. USA (1997) vol. 94, p. 12297-12302. Alternatively, it may be a DNA-protein library (for example, as described in Lohse, DNA-Protein Fusions and Uses Thereof, U.S.S.N. 60/110,549, filed December 2, 1998 and _ , filed December 2, 1999). The fusion library is incubated with the immobilized target, the support is washed to remove non-specific binders, and the tightest binders are eluted under very stringent conditions and subjected to PCR to recover the sequence information or to create a new library of binders which may be used to repeat the selection process, with or without further mutagenesis of the sequence. A number of rounds of selection may be performed until binders of sufficient affinity for the antigen are obtained.

In one particular example, the ¹⁰Fn3 scaffold may be used as the selection target. For example, if a protein is required that binds a specific peptide sequence presented in a ten residue loop, a single ¹⁰Fn3 clone is constructed in which one of its loops has been set to the length of ten and to the desired sequence. The new clone is expressed *in vivo* and purified, and then immobilized on a solid support. An RNA-protein fusion library based on an appropriate scaffold is then allowed to interact with the support, which is then washed, and desired molecules eluted and re-selected as described above.

Similarly, the ¹⁰Fn3 scaffold may be used to find natural proteins that interact with the peptide sequence displayed in a ¹⁰Fn3 loop. The ¹⁰Fn3 protein is immobilized as described above, and an RNA-protein fusion library is screened for binders to the displayed loop. The binders are enriched through multiple rounds of selection and identified by DNA sequencing.

In addition, in the above approaches, although RNA-protein libraries represent exemplary libraries for directed evolution, any type of scaffold-based library may be used in the selection methods of the invention.

### Use

The antibody mimics described herein may be evolved to bind any antigen of interest. These proteins have thermodynamic properties superior to those of natural antibodies and can be evolved rapidly in vitro. Accordingly, these antibody mimics may be employed in place of antibodies in all areas in which antibodies are used, including in the research, therapeutic, and diagnostic fields. In addition, because these scaffolds possess solubility and stability properties superior to antibodies, the antibody mimics described herein may also be used under conditions which would destroy or inactivate antibody molecules. Finally, because the scaffolds of the present invention may be evolved to bind virtually any compound, these molecules provide completely novel binding proteins which also find use in the research, diagnostic, and therapeutic areas.

### Experimental Results

Exemplary scaffold molecules described above were generated and tested, for example, in selection protocols, as follows.

### Library construction

A complex library was constructed from three fragments, each of which contained one randomized area corresponding to a CDR-like loop. The fragments were named BC, DE, and FG, based on the names of the CDR-H-like loops contained within them; in addition to ¹⁰Fn3 and a randomized sequence, each of the fragments contained stretches encoding an N-terminal His₆ domain or a C-terminal FLAG peptide tag. At each junction between two fragments (i.e., between the BC and DE fragments or between the DE and FG fragments), each DNA fragment contained recognition sequences for the EarI Type IIS restriction endonuclease. This restriction enzyme allowed the splicing together of adjacent fragments while removing all foreign, non-¹⁰Fn3, sequences. It also allows for a recombination-like mixing of the three ¹⁰Fn3 fragments between cycles of mutagenesis and selection.

Each fragment was assembled from two overlapping oligonucleotides, which were first annealed, then extended to form the double-stranded DNA form of the fragment. The oligonucleotides that were used to construct and process the three fragments are listed below; the "Top" and "Bottom" species for each fragment are the oligonucleotides that contained the entire ¹⁰Fn3 encoding sequence. In these oligonucleotides designations, "N" indicates A, T, C, or G; and "S" indicates C or G.
**HfnLbcTop (His):** 
5'- GG AAT TCC TAA TAC GAC TCA CTA TAG GGA CAA TTA CTA
TTT ACA ATT ACA ATG CAT CAC CAT CAC CAT CAC GTT TCT GAT
GTT CCG AGG GAC CTG GAA GTT GTT GCT GCG ACC CCC ACC
AGC-3' (SEQ ID NO: 1)
 **HfnLbcTop (an alternative N-terminus):** 
5'- GG AAT TCC TAA TAC GAC TCA CTA TAG GGA CAA TTA CTA
TTT ACA ATT ACA ATG GTT TCT GAT GTT CCG AGG GAC CTG GAA
GTT GTT GCT GCG ACC CCC ACC AGC-3' (SEQ ID NO: 2)
 **HFnLBCBot-flag8:** 
5'-AGC GGA TGC CTT GTC GTC GTC GTC CTT GTA GTC GCT CTT
CCC TGT TTC TCC GTA AGT GAT CCT GTA ATA TCT (SNN)7 CCA
GCT GAT CAG TAG GCT GGT GGG GGT CGC AGC -3' (SEQ ID NO: 3)
 **HFnBC3'-flag8:** 
5'-AGC GGA TGC CTT GTC GTC GTC GTC CTT GTA GTC GCT CTT
CCC TGT TTC TCC GTA AGT GAT CC-3' (SEQ ID NO: 4)
 **HFnLDETop:** 
5'- GG AAT TCC TAA TAC GAC TCA CTA TAG GGA CAA TTA CTA
TTT ACA ATT ACA ATG CAT CAC CAT CAC CAT CAC CTC TTC ACA
GGA GGA AAT AGC CCT GTC C-3' (SEQ ID NO: 5)
 **HFnLDEBot-flag8:** 
5'-AGC GGA TGC CTT GTC GTC GTC GTC CTT GTA GTC GCT CTT
CGT ATA ATC AAC TCC AGG TTT AAG GCC GCT GAT GGT AGC TGT
(SNN)4 AGG CAC AGT GAA CTC CTG GAC AGG GCT ATT TCC TCC
TGT -3' (SEQ ID NO: 6)
 **HFnDE3'-flag8:** 
5'-AGC GGA TGC CTT GTC GTC GTC GTC CTT GTA GTC GCT CTT
CGT ATA ATC AAC TCC AGG TTT AAG G-3' (SEQ ID NO: 7)
 **HFnLFGTop:** 
5'- GG AAT TCC TAA TAC GAC TCA CTA TAG GGA CAA TTA CTA
TTT ACA ATT ACA ATG CAT CAC CAT CAC CAT CAC CTC TTC TAT
ACC ATC ACT GTG TAT GCT GTC-3' (SEQ ID NO: 8)
 **HFnLFGBot-flag8:** 
5'-AGC GGA TGC CTT GTC GTC GTC GTC CTT GTA GTC TGT TCG
GTA ATT AAT GGA AAT TGG (SNN)10 AGT GAC AGC ATA CAC AGT
GAT GGT ATA -3' (SEQ ID NO: 9)
 **HFnFG3'-flag8:** 
5'-AGC GGA TGC CTT GTC GTC GTC GTC CTT GTA GTC TGT TCG
GTA ATT AAT GGA AAT TGG -3' (SEQ ID NO: 10)
 **T7Tmv (introduces T7 promoter and TMV untranslated region needed for in vitro translation):** 
5'- GCG TAA TAC GAC TCA CTA TAG GGA CAA TTA CTA TTT ACA
ATT ACA-3' (SEQ ID NO: 11)
 **ASAflag8:** 
5'-AGC GGA TGC CTT GTC GTC GTC GTC CTT GTA GTC-3' (SEQ ID
NO: 12)
 **Unispl-s (spint oligonucleotide used to ligate mRNA to the puromycin-containing linker, described by Roberts et al, 1997, supra):**
5'-TTTTTTTTTNAGCGGATGC-3' (SEQ ID NO: 13)
**A18--2PEG (DNA-puromycin linker):**
5'-(A) 18(PEG)2CCPur (SEQ ID NO: 14)

The pairs of oligonucleotides (500 pmol of each) were annealed in 100 µL of 10 mM Tris 7.5, 50 mM NaCl for 10 minutes at 85°C, followed by a slow (0.5-1 hour) cooling to room temperature. The annealed fragments with single-stranded overhangs were then extended using 100 U Klenow (New England Biolabs, Beverly, MA) for each 100 µL aliquot of annealed oligos, and the buffer made of 838.5 µl H₂O, 9 µl 1 M Tris 7.5, 5 µl 1M MgCl₂, 20 µl 10 mM dNTPs, and 7.5 µl 1M DTT. The extension reactions proceeded for 1 hour at 25°C.

Next, each of the double-stranded fragments was transformed into a RNA-protein fusion (PROfusion^{™}) using the technique developed by Szostak et al., U.S.S.N. 09/007,005 and U.S.S.N. 09/247,190; Szostak et al., WO98/31700; and Roberts & Szostak, Proc. Natl. Acad. Sci. USA (1997) vol. 94, p. 12297-12302. Briefly, the fragments were transcribed using an Ambion in vitro transcription kit, MEGAshortscript (Ambion, Austin, TX), and the resulting mRNA was gel-purified and ligated to a DNA-puromycin linker using DNA ligase. The mRNA-DNA-puromycin molecule was then translated using the Ambion rabbit reticulocyte lysate-based translation kit. The resulting mRNA-DNA-puromycin-protein PROfusion^{™} was purified using Oligo(dT) cellulose, and a complementary DNA strand was synthesized using reverse transcriptase and the RT primers described above (Unisplint-S or flagASA), following the manufacturer's instructions.

The PROfusion^{™} obtained for each fragment was next purified on the resin appropriate to its peptide purification tag, i.e., on Ni-NTA agarose for the His₆-tag and M2 agarose for the FLAG-tag, following the procedure recommended by the manufacturer. The DNA component of the tag-binding PROfusions^{™} was amplified by PCR using Pharmacia Ready-to-Go PCR Beads, 10 pmol of 5' and 3' PCR primers, and the following PCR program (Pharmacia, Piscataway, NJ): Step 1: 95°C for 3 minutes; Step 2: 95°C for 30 seconds, 58/62°C for 30 seconds, 72°C for 1 minute, 20/25/30 cycles, as required; Step 3: 72°C for 5 minutes; Step 4: 4°C until end.

The resulting DNA was cleaved by 5 U EarI (New England Biolabs) perl ug DNA; the reaction took place in T4 DNA Ligase Buffer (New England Biolabs) at 37°C, for 1 hour, and was followed by an incubation at 70°C for15 minutes to inactivate Ear I. Equal amounts of the BC, DE, and FG fragments were combined and ligated to form a full-length ¹⁰Fn3 gene with randomized loops. The ligation required 10 U of fresh EarI (New England Biolabs) and 20 U of T4 DNA Ligase (Promega, Madison, WI), and took 1 hour at 37°C.

Three different libraries were made in the manner described above. Each contained the form of the FG loop with 10 randomized residues. The BC and the DE loops of the first library bore the wild type ¹⁰Fn3 sequence; a BC loop with 7 randomized residues and a wild type DE loop made up the second library; and a BC loop with 7 randomized residues and a DE loop with 4 randomized residues made up the third library. The complexity of the FG loop in each of these three libraries was 10¹³; the further two randomized loops provided the potential for a complexity too large to be sampled in a laboratory.

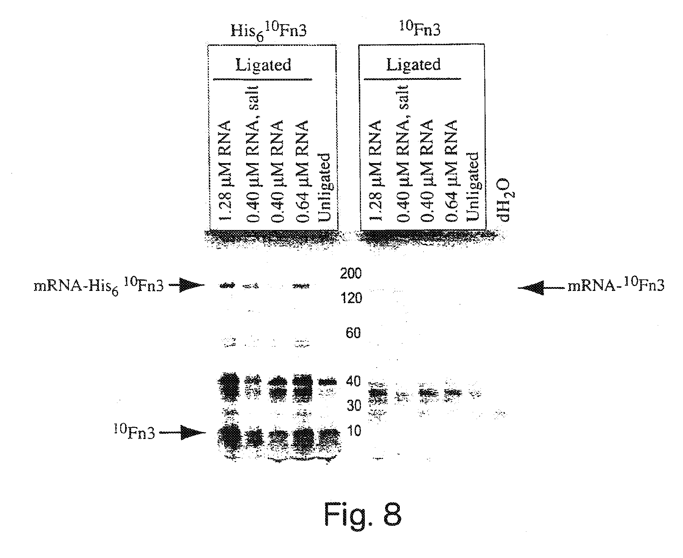

The three libraries constructed were combined into one master library in order to simplify the selection process; target binding itself was expected to select the most suitable library for a particular challenge. PROfusions^{™} were obtained from the master library following the general procedure described in Szostak et al., U.S.S.N. 09/007,005 and 09/247,190; Szostak et al., WO98/31700; and Roberts & Szostak, Proc. Nat1. Acad. Sci. USA (1997) vol. 94, p. 12297-12302 (Figure 8).

### Fusion Selections

The master library in the PROfusion^{™} form was subj ected to selection for binding to TNF-α. Two protocols were employed: one in which the target was immobilized on an agarose column and one in which the target was immobilized on a BIACORE chip. First, an extensive optimization of conditions to minimize background binders to the agarose column yielded the favorable buffer conditions of 50 mM HEPES pH 7.4, 0.02% Triton, 100 µg/ml Sheared Salmon Sperm DNA. In this buffer, the non-specific binding of the ¹⁰Fn3 RNA fusion to TNF-α Sepharose was 0.3%. The non-specific binding background of the ¹⁰Fn3 RNA-DNA to TNF-α Sepharose was found to be 0.1%.

During each round of selection on TNF-α Sepharose, the Profusion^{™} library was first preincubated for an hour with underivatized Sepharose to remove any remaining non-specific binders; the flow-through from this pre-clearing was incubated for another hour with TNF-α Sepharose. The TNF-α Sepharose was washed for 3-30 minutes.

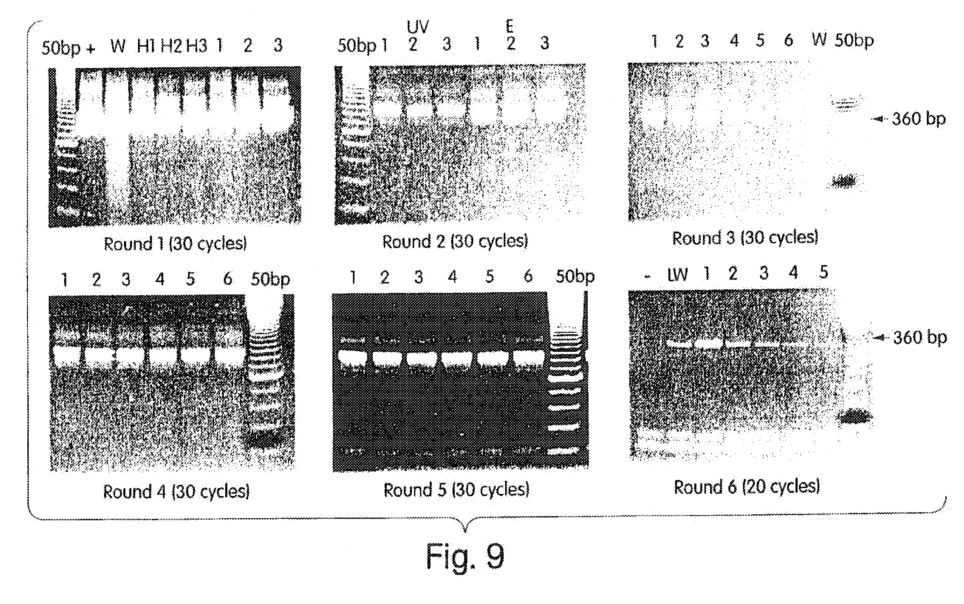

After each selection, the PROfusion^{™} DNA that had been eluted from the solid support with 0.3 M NaOH or 0.1M KOH was amplified by PCR; a DNA band of the expected size persisted through multiple rounds of selection (Figure 9); similar results were observed in the two alternative selection protocols, and only the data from the agarose column selection is shown in Figure 9.

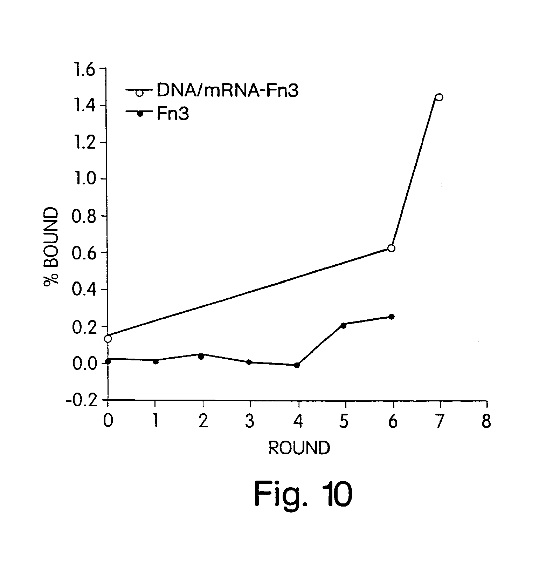

In the first seven rounds, the binding of library PROfusions^{™} to the target remained low; in contrast, when free protein was translated from DNA pools at different stages of the selection, the proportion of the column binding species increased significantly between rounds (Figure 10). Similar selections may be carried out with any other binding species target (for example, IL-1 and IL-13).

### Animal Studies

Wild-type ¹⁰Fn3 contains an integrin-binding tripepetide motif, Arginine 78 - Glycine 79 - Aspartate 80 (the "RGD motif) at the tip of the FG loop. In order to avoid integrin binding and a potential inflammatory response based on this tripeptide in vivo, a mutant form of ¹⁰Fn3 was generated that contained an inert sequence, Serine 78 - Glycine 79 - Glutamate 80 (the "SGE mutant"), a sequence which is found in the closely related, wild-type "Fn3 domain. This SGE mutant was expressed as an N-terminally His₆-tagged, free protein in E. coli, and purified to homogeneity on a metal chelate column followed by a size exclusion column.

In particular, the DNA sequence encoding His₆-¹⁰Fn3(SGE) was cloned into the pET9a expression vector and transformed into BL21 DE3 pLysS cells. The culture was then grown in LB broth containing 50 µg/mL kanamycin at 37°C, with shaking, to A₅₆₀=1.0, and was then induced with 0.4 mM IPTG. The induced culture was further incubated, under the same conditions, overnight (14-18 hours); the bacteria were recovered by standard, low speed centrifugation. The cell pellet was resuspended in 1/50 of the original culture volume of lysis buffer (50 mM Tris 8.0, 0.5 M NaCl, 5% glycerol, 0.05% Triton X-100, and 1 mM PMSF), and the cells were lysed by passing the resulting paste through a Microfluidics Corporation Microfluidizer M110-EH, three times. The lysate was clarified by centrifugation, and the supernatant was filtered through a 0.45 µm filter followed by filtration through a 0.2 µm filter. 100 mL of the clarified lysate was loaded onto a 5 mL Talon cobalt column (Clontech, Palo Alto, CA), washed by 70 mL of lysis buffer, and eluted with a linear gradient of 0-30 mM imidazole in lysis buffer. The flow rate through the column through all the steps was 1 mL/min. The eluted protein was concentrated 10-fold by dialysis (MW cutoff = 3,500) against 15,000-20,000 PEG. The resulting sample was dialysed into buffer 1 (lysis buffer without the glycerol), then loaded, 5 mL at a time, onto a 16 x 60 mm Sephacryl 100 size exclusion column equilibrated in buffer 1. The column was run at 0.8 mL/min, in buffer 1; all fractions that contained a protein of the expected MW were pooled, concentrated 10X as described above, then dialyzed into PBS. Toxikon (MA) was engaged to perform endotoxin screens and animal studies on the resulting sample.

In these animal studies, the endotoxin levels in the samples examined to date have been below the detection level of the assay. In a preliminary toxicology study, this protein was injected into two mice at the estimated 100X therapeutic dose of 2.6 mg/mouse. The animals survived the two weeks of the study with no apparent ill effects. These results suggest that ¹⁰Fn3 may be incorporated safely into an IV drug.

### Alternative Constructs for In Vivo Use

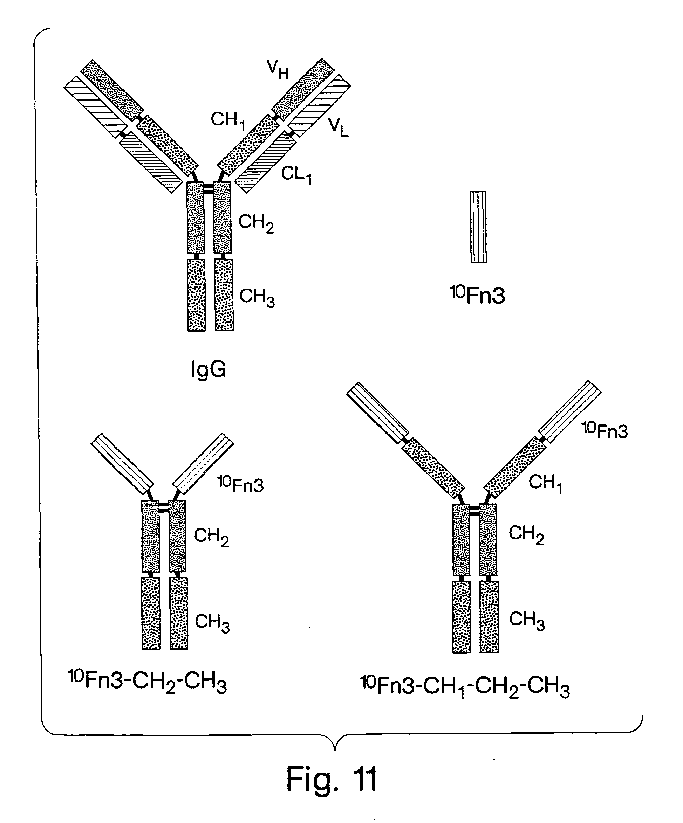

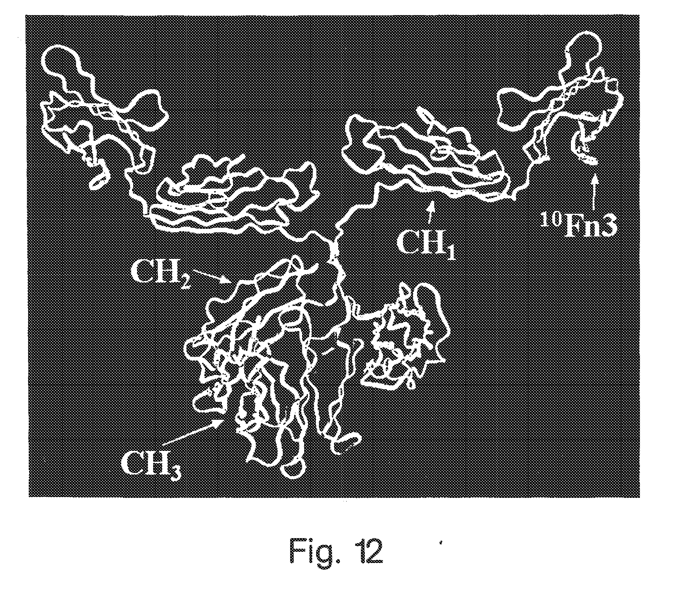

To extend the half life of the 8 kD ¹⁰Fn3 domain, a larger molecule has also been constructed that mimics natural antibodies. This ¹⁰Fn3-F_{c} molecule contains the -CH₁-CH₂-CH₃ (Figure 11) or -CH₂-CH₃ domains of the IgG constant region of the host; in these constructs, the ¹⁰Fn3 domain is grafted onto the N-terminus in place of the IgG V_{H} domain (Figures 11 and 12). Such antibody-like constructs are expected to improve the pharmacokinetics of the protein as well as its ability to harness the natural immune response.

In order to construct the murine form of the ¹⁰Fn3-CH₁-CH₂-CH₃ clone, the -CH₁-CH₂-CH₃ region was first amplified from a mouse liver spleen cDNA library (Clontech), then ligated into the pET25b vector. The primers used in the cloning were 5' Fc Nest and 3' 5 Fc Nest, and the primers used to graft the appropriate restriction sites onto the ends of the recovered insert were 5' Fc HIII and 3' Fc Nhe:
5' FG Nest 5'GCG GCA GGG TTT GCT TAC TGG GGC CAA GGG 3' (SEQ ID NO: 15);
3' Fc Nest 5'GGG AGG GGT GGA GGT AGG TCA CAG TCC 3' (SEQ ID NO: 16);
3' Fc Nhe 5' TTT GCT AGC TTT ACC AGG AGA GTG GGA GGC 3' (SEQ ID NO: 17); and
5' Fc HIII 5' AAA AAG CTT GCC AAA ACG ACA CCC CCA TCT GTC 3' (SEQ ID NO: 18).

Further PCR is used to remove the CH₁ region from this clone and create the Fc part of the shorter, ¹⁰Fn3-CH₂-CH₃ clone. The sequence encoding ¹⁰Fn3 is spliced onto the 5' end of each clone; either the wild type ¹⁰Fn3 cloned from the same mouse spleen cDNA library or a modified ¹⁰Fn3 obtained by mutagenesis or randomization of the molecules can be used. The oligonucleotides used in the cloning of murine wild-type ¹⁰Fn3 were:
Mo 5PCR-NdeI:
   5' CATATGGTTTCTGATATTCCGAGAGATCTGGAG 3' (SEQ ID NO: 19);
Mo5PCR-His-NdeI (for an alternative N-terminus with the His₆ purification tag): 
5' CAT ATG CAT CAC CAT CAC CAT CAC GTT TCT GAT ATT
CCG AGA G 3' (SEQ ID NO: 20);
 and
Mo3PCR-EcoRI: 5'
   GAATTCCTATGTTTTATAATTGATGGAAAC3' (SEQ ID NO: 21).

The human equivalents of the clones are constructed using the same strategy with human oligonucleotide sequences.

Other embodiments are within the claims.

All publications, patents, and patent applications mentioned herein are hereby incorporated by reference.

## Claims

1. A protein comprising a tenth fibronectin type III domain (¹⁰Fn3) wherein the amino acid sequence of at least one loop selected from the group consisting of the BC loop, the DE loop and the FG loop comprises one or more amino acid alterations relative to the sequence of a naturally occurring human tenth fibronectin type III domain, said protein being **characterized by** its ability to bind to a compound.

2. The protein of claim 1, wherein the amino acid sequence of the naturally occurring human ¹⁰Fn3 is set forth in figure 4.

3. The protein of claim 2, wherein the BC loop of the naturally occurring human ¹⁰Fn3 runs between amino acid residues 21-31 of the sequence as set forth in figure 4; the DE loop of the naturally occurring human ¹⁰Fn3 runs between amino acid residues 51-56 of the sequence as set forth in figure 4; and the FG loop of the naturally occurring human ¹⁰Fn3 runs between amino acid residues 76-88 of the sequence as set forth in figure 4.

4. The protein of any one of claims 1 to 3, wherein the amino acid sequence of each of the BC loop, the DE loop and the FG loop comprises one or more amino acid alterations relative to the sequence of the naturally occurring human tenth fibronectin type III domain.

5. The protein of any one of claims 1 to 3, wherein the DE loop of said ¹⁰Fn3 is extended in length relative to the naturally occurring human ¹⁰Fn3.

6. The protein of any one of claims 1 to 5, wherein said ¹⁰Fn3 lacks an integrin-binding motif.

7. The protein of any one of claims 1 to 6, wherein said protein is covalently bound to a nucleic acid and said nucleic acid encodes said protein.

8. The protein of claim 7, wherein said nucleic acid is RNA.

9. A fusion protein comprising the protein of any one of claims 1 to 8.

10. A composition comprising the protein of any one of claims 1 to 8 and a physiologically acceptable carrier.

11. A protein of any one of claims 1 to 8 for use in therapy or diagnosis.

12. A nucleic acid encoding the protein of any one of claims 1 to 8.

13. A method for obtaining a protein of any one of claims 1 to 8, said method comprising:
(a) randomizing at least one loop selected from the group consisting of the BC loop, the DE loop and the FG loop of a tenth fibronectin type III domain (¹⁰Fn3) thereby creating a library of proteins comprising a tenth fibronectin type III domain wherein the amino acid sequence of the loop comprises one or more amino acid alterations relative to the sequence of a naturally occurring human tenth fibronectin type III domain;
(b) contacting a compound with the proteins, said contacting being carried out under conditions that allow compound-protein complex formation;
(c) selecting a compound-protein complex; and
(d) obtaining, from said complex, said protein which binds to said compound.

14. The method of claim 13, said method further comprising randomizing at least one loop of said protein obtained in step (d) and repeating said steps (b), (c) and (d) using said further randomized protein.

15. A method for obtaining a compound which binds to a protein of any one of claims 1 to 8, said method comprising:
(a) contacting said protein with a candidate compound, said contacting being carried out under conditions that allow compound-protein complex formation; and
(b) obtaining, from said complex, said compound which binds to said protein.
